# FASCICULE DE BREVET EUROPEEN

(11) **EP 0 865 777 B1**
(45) Date de publication et mention de la délivrance du brevet: **25.09.2002**
(21) Numéro de dépôt: 98420017.0
(22) Date de dépôt: 28.01.1998
(51) Int. Cl.: A61F 2/36

(54) **Tige fémorale pour prothèse de hanche**
Femurschaftprothese für die Hüfte
Femoral stem for a hip prosthesis

(30) Priorité: 21.03.1997 FR 9703725
(43) Date de publication de la demande: 23.09.1998
(73) Titulaire: Biomet Merck France, 26000 Valence (FR); ORA, 69160 Tassin la Demi Lune (FR)
(72) Inventeur: Augoyard, Marc, 69005 Lyon (FR); Bascoulergue, Gérard, 62520 Le Touquet (FR); Basso, Maurice, 43000 Le Puy (FR); Benedetto, Murielle, 26000 Valence (FR); Bertocchi, René, 69006 Lyon (FR); Charret, Philippe, 69270 Fontaines Sur Saone (FR); Courcelles, Philippe, 69570 Dardilly (FR); Debiesse, Jean-Louis, Montée Coupe Jarrets, 38200 Vienne (FR); Dupre-Latour, Laurent, 42580 L'Etrat (FR); Eyraud, Guy, 38150 Peage du Roussillon (FR); Fayard, Jean-Philippe, 42170 Saint Just Saint Rambert (FR); Hulin, Paul Henri, 89000 Auxerre (FR); Lecuire, François, Giens, 83400 Hyeres (FR); Melere, Gilles, 74000 Annecy (FR); Millon, Joseph, 73490 La Ravoire (FR); Passot, Jean-Paul, 42530 St Genest Lerpt (FR); Peyrot, Jacques, 69005 Lyon (FR); Relave, Marc, 42580 L'Etrat (FR); De Witte, Gérard, 26300 Chateauneuf Sur Isere (FR)
(74) Mandataire: Vuillermoz, Bruno

(56) Documents cités:
- EP-A- 0 485 311
- EP-A- 0 695 540
- FR-A- 2 637 494
- FR-A- 2 680 315

## Description

L'invention concerne une nouvelle tige fémorale pour prothèse de hanche.

De manière connue, une prothèse de hanche est constituée d'une tige fémorale, destinée à être insérée dans le canal médullaire du fémur de l'articulation de la hanche considérée, et un col prothétique, surmonté d'une tête, faisant ou non partie intégrante de la tige, et destiné à coopérer à son extrémité avec un noyau de frottement, le plus souvent réalisé en polyéthylène. Ce dernier est destiné à coopérer avec la cavité cotyloïdienne de l'os iliaque de la hanche considérée, au niveau de laquelle est préalablement inséré ou mis en place un cotyle de forme appropriée.

Parmi les nombreuses difficultés rencontrées avec ces prothèses, on peut citer les descellements de la tige fémorale hors du fémur, mais également les pics de contrainte exercés au niveau, notamment de la corticale interne du fémur par la tige, et se traduisant par des douleurs pour le patient. Afin de s'affranchir de ces pics de contrainte, on cherche autant que faire se peut, à rendre le plus anatomique possible les tiges fémorales en question.

On a ainsi proposé, par exemple dans le document EP-A-0 485 311, une prothèse à antéversion modulable, dans lequel le col prothétique est rapporté au niveau de la portion proximale de la tige fémorale, après mise en place de celle-ci au sein du fémur à prothéser, l'angle d'antéversion étant choisi en per-opératoire par le praticien lors de l'intervention, afin d'adapter au mieux la prothèse à la morphologie et à l'anatomie du patient.

Cette antéversion modulable permet d'optimiser la recherche de l'anatomie, notamment au niveau de l'articulation proprement dite, et permet également de diminuer notablement les risques de luxation. Néanmoins, elle ne permet pas de régler le problème du caractère anatomique de la tige, puisqu'il n'est pas tenu compte de l'antéflexion et de la courbe sagittale du fémur, ni du caractère hélitorsé de la métaphyse proximale fémorale.

On a de fait proposé dans le document EP-A-0 695 540, une prothèse de hanche dont le profil général de la tige présente une antétorsion se développant sensiblement de la zone métaphyso-diaphysaire à la zone épiphysaire de la tige. Par ailleurs, la tige est incurvée notamment au niveau de sa zone proximale selon un profil hélitorsé par rapport à l'axe longitudinal passant par le centre de l'extrémité inférieure de la zone distale de la tige et s'étendant en direction de la phase interne de la tige, celle-ci présentant au niveau de sa zone proximale une rotation partielle sur elle-même se développant sensiblement de la zone métaphyso-diaphysaire à la zone épiphysaire.

De par l'augmentation de l'adaptation anatomique de la tige au fémur, on optimise la solidité de sa mise en place au niveau du fémur.

Pour autant, si les tiges correspondant à ce profil donnent satisfaction, on a souhaité optimiser la fixation proprement dite de celles-ci, notamment au niveau de la zone métaphysaire afin, d'une part, d'assurer une certaine uniformité de la répartition des contraintes au niveau de cette section métaphysaire pour promouvoir la mise sous contrainte de l'os à ce niveau et partant, la fixation primaire, et d'autre part, de s'affranchir de tout risque de pression de ladite tige au niveau de sa zone distale contre la corticale interne du fémur, afin de limiter voir annuler les douleurs supportées par le patient.

Cette optimisation de la fixation de la prothèse de hanche est obtenue par la tige fémorale conforme à l'invention. Celle-ci concerne donc une tige fémorale destinée à être enfouie dans le fémur à prothéser, se prolongeant par un col prothétique, dont l'extrémité est destinée à recevoir une tête destinée à coopérer avec un noyau de frottement lui-même reçu dans la cavité cotyloïdienne de l'os iliaque de l'articulation coxo-fémorale considérée. Elle se caractérise en ce que la partie métaphysaire de la tige présente au niveau de sa partie antérieure, définie classiquement comme étant la face dirigée vers le devant du patient, lorsque la prothèse est en place, une excroissance, de telle sorte à davantage remplir et combler le défaut de matière osseuse à ce niveau, lors de la mise en place de la tige.

En d'autres termes, l'invention consiste à conférer à la tige une forme telle, qu'on aboutit à un blocage métaphysaire trois points, de telle sorte à optimiser la fixation primaire de la tige au niveau du fémur. De la même manière, l'invention consiste à conférer une asymétrie dans le plan sagittal à l'axe du col prothétique par rapport à son embase. Ce faisant, on optimise le remplissage de la partie vide du fémur, et partant, on améliore sensiblement la fixation de la prothèse.

Selon l'invention, l'excroissance développée par la tige va en s'élargissant d'une part de la zone métaphyso-diaphysaire en direction de la zone épiphysaire de la tige, et d'autre part de la zone corticale interne vers la face postéro-externe de ladite tige.

Selon une caractéristique de l'invention, la tige présente au niveau de sa face postéro-externe une angulation, dont le sommet est aligné avec l'axe du col prothétique.

Cette caractéristique additionnée à la caractéristique précédente renforce la répartition uniforme des contraintes au niveau de la zone métaphysaire et partant, la fixation primaire elle-même de la tige.

Avantageusement, le col de la tige présente également une antéversion de l'ordre de 8°, résultant d'une hélitorsion progressive de la partie métaphysaire à partir de la jonction métaphyso-diaphysaire.

La tige présente également une courbure dans le plan sagittal, selon un rayon moyen voisin de 1400 mm, s'étendant également sensiblement de la jonction métaphyso-diaphysaire en direction de la zone épiphysaire.

La tige présente sur chacune de ses deux faces principales des rainures, non débouchantes au niveau de l'extrémité supérieure, c'est à dire la zone proximale, et se terminant à leur extrémité inférieure par un rayon important, typiquement supérieur à 1200 mm, de telle sorte à faciliter l'extraction de la tige le cas échéant.

La manière dont l'invention peut être réalisée et les avantages qui en découlent ressortiront mieux de l'exemple de réalisation qui suit, donné à titre indicatif et non limitatif, à l'appui des figures annexées.

La figure 1 est une vue schématique de face de la tige selon l'invention.

La figure 2 est une représentation schématique représentant une vue du dessous de la tige conforme à l'invention.

La figure 3 est une représentation schématique représentant une vue du dessus de la tige conforme à l'invention.

La figure 4 est une autre représentation schématique de face de la tige selon l'invention, dont les figures 5 et 6 sont des vues dans le plan sagittal et la figure 7, une vue du dessus.

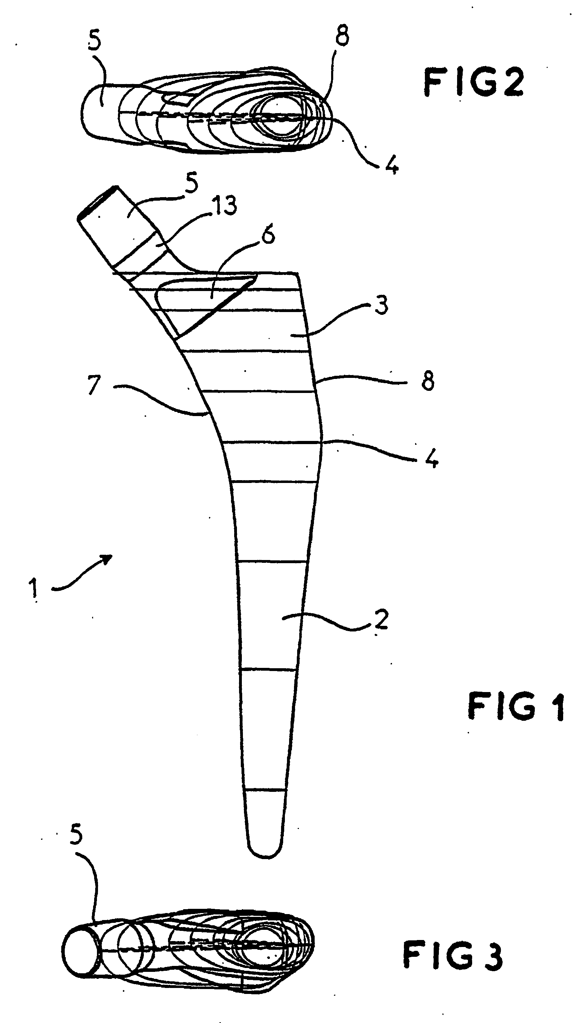

La figure 1 représente schématiquement, ainsi que déjà dit, une tige fémorale (1) pour prothèse de hanche conforme à l'invention, se prolongeant à son extrémité proximale par un col prothétique (5). De manière connue, le col prothétique (5) est destiné à dépasser du fémur à prothéser et comprend dans le cas d'espèce un cône morse destiné à recevoir une tête sphérique, le plus souvent réalisée en acier inoxydable ou en céramique, et destinée à son tour à coopérer avec la cupule cotyloïdienne par l'intermédiaire d'un noyau de frottement.

Traditionnellement, la face interne (7) de la tige présente une courbure, ici selon un rayon de 1500 mm, correspondant sensiblement à la courbure moyenne du fémur.

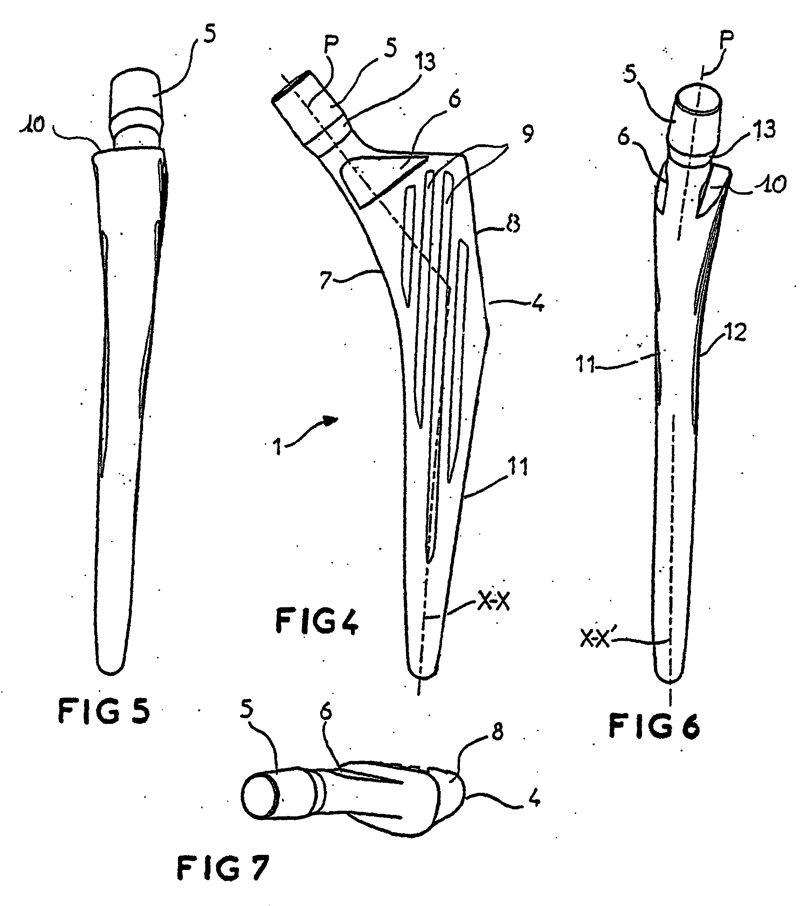

La face externe (8) est constituée de deux parties sensiblement rectilignes, concourantes entre elles au niveau d'un épaulement (4), qui selon l'une des caractéristiques de l'invention, est située dans le prolongement de l'axe P du col prothétique (5) ainsi, par ailleurs, que cela apparaît très nettement sur la figure 4.

Le positionnement particulier de cet épaulement, dans le prolongement du col prothétique, optimise le bon centrage métaphysaire de la tige au sein du canal médullaire, et le respect du bras de levier des muscles fessiers. En effet, afin de respecter ce bras de levier, il importe de respecter la distance entre la fossette digitale du fémur et le centre de la tête dudit fémur. En d'autres termes, compte tenu du remplacement de la partie supérieure du fémur à prothéser, il importe que la prothèse mise en place satisfasse autant que faire se peut ces caractéristiques. Or, les tailles croissantes de la tige fémorale des prothèses proposées vont généralement à l'inverse de ce résultat. En revanche, de par la géométrie conférée à la tige conforme à l'invention, on respecte ces caractéristiques, tout en maintenant constant l'angle cervico-diaphysaire.

De par cette forme particulière de la partie métaphysaire (3) de la tige, on aboutit à un fixation trois points de celle-ci au niveau de cette zone. Par ce biais, on limite le remodelage osseux lors de la mise en place de la tige, et partant, on conserve davantage de capital osseux. Corollairement, les risques de descellement sont diminués. De plus, on aboutit à une homogénéisation des contraintes au niveau de cette zone métaphysaire, fiabilisant dans le temps la fixation de la prothèse au niveau du fémur prothésé.

Selon une caractéristique essentielle de l'invention, la tige présente une excroissance (10) (figure 5) se développant sensiblement de la jonction médiaphysodiaphysaire jusqu'à la zone épiphysaire, et de la corticale interne en direction de la corticale externe du fémur, et propre à augmenter le remplissage métaphysaire de face et de profil au niveau de la base du col du fémur alors ôté. Ce faisant, on aboutit à une optimisation du centrage de la tige dans le canal médullaire, et corollairement à un meilleur ancrage de celle-ci au sein du fémur prothésé. Par ailleurs, de par ce meilleur centrage, on s'affranchit ou on diminue fortement, les risques de mise sous contrainte de la corticale interne du fémur en portion diaphysaire.

Du fait de cette excroissance, le col prothétique (5) se trouve excentré vers l'arrière par rapport à l'axe longitudinal de la dernière section transversale représentée sur la figure 1. En effet, on a représenté au sein de la figure 1, et corollairement sur les figures 2 et 3, différentes sections transversales effectuées perpendiculairement par rapport à l'axe X-X de la tige, c'est à dire par rapport à l'axe de symétrie de la zone distale (2) de la tige.

Ces différentes sections rapportées sur les figures 2 et 3 mettent en évidence l'hélitorsion de la tige, aboutissant à une antéversion du col prothétique (5) de l'ordre de 8°.

Cette notion d'hélitorsion et d'antéversion a été largement décrite dans la demande EP-A-0 695 540 du Demandeur, déjà citée, de sorte qu'il n'y a pas lieu de s'y appesantir en détail. Il convient néanmoins de rappeler que le profil de la tige présente une antétorsion entre la zone diaphysaire et plus précisément métaphyso-diaphysaire, c'est à dire centrale, jusqu'à la zone supérieure proximale de la tige. Cette antétorsion correspond d'une part, à une incurvation de la tige au niveau d'au moins l'une de ses faces latérales, et d'autre part, à une torsion hélicoïdale dans sa partie proximale supérieure en direction de la droite ou de la gauche, selon le fémur à prothéser droit ou gauche.

Selon une autre caractéristique de l'invention, la tige fémorale selon l'invention comporte sur ses faces principales (11) et (12) des rainures (9) adaptées au profil de la tige. Ces rainures (9) présentent une profondeur voisine et constantes de 1,5 mm, et sont non débouchantes au niveau de la partie proximale de la tige. De la sorte, on s'affranchit des risques de migration de micro-particules, telles que par exemple du ciment, à l'interface os - prothèse ou os - ciment - prothèse, dont on a pu montrer qu'ils pouvaient être à l'origine de réactions macrophagiques, entraînant la disparition par lyse de l'os, et corollairement descellement de la prothèse.

Selon une caractéristique de l'invention, ces rainures (9) sont d'une profondeur . constante le long du profil de la tige, évitant ainsi l'uniformisation de l'ancrage osseux.

Par ailleurs, l'extrémité inférieure de ces rainures est fuyante vers le bas et présentant notamment un rayon important, typiquement supérieur à 1200 mm, de telle sorte à faciliter le cas échéant l'extraction de la tige.

Selon l'invention, la courbure de la face interne (7) de la tige présente un rayon de l'ordre de 1500 mm, correspondant à la courbure moyenne du fémur. De plus, la section transversale de l'extrémité distale de la tige est circulaire et se prolonge par une section sensiblement elliptique.

Le col prothétique (5) forme avec l'axe de la tige un angle cervico-diaphysaire voisin de 137,5 °. Il présente à sa base deux plats latéraux symétriques (6), permettant une augmentation du débattement angulaire. Par ailleurs, il présente un rétreint (13) à la base de sa partie conique, au niveau de la zone de liaison avec les deux plats latéraux (6).

Typiquement, ce rétreint (13) présente à sa base un diamètre de 11,5 mm, alors que la base du cone morse (5) présente un diamètre de 14 mm.

De manière traditionnelle, la tige conforme à l'invention, est réalisée en acier inoxydable, voire en alliage de titane et d'aluminium. Selon le mode de fixation de la tige, notamment si celle-ci doit être mise en place sans ciment, la tige est recouverte d'hydroxyapatite de calcium.

Elle peut être utilisée indifféremment comme implant céphalique, en cas de fracture du col du fémur, ou être intégrée dans un système de prothèse totale de hanche, comprenant dans ce cas une tige fémorale, une tête fémorale et une prothèse acétabulaire.

La tige fémorale conforme à l'invention présente, par rapport à la précédente, de nombreux avantages, parmi lesquels le plus important concerne l'optimisation de la fixation de la tige, notamment de la zone métaphysaire, optimisation renforcée par une augmentation de remplissage de ladite partie métaphysaire augmentant le contact de la tige vers l'avant.

Cette augmentation du remplissage optimise en outre l'ancrage de la tige, et partant réduit les risques de descellement. Qui plus est et comme déjà dit, il améliore le centrage, de sorte qu'on évite les zones de contrainte de la partie distale de la tige au niveau de la corticale interne du fémur et partant, les risques de douleurs.

## Revendications

1. Tige fémorale (1) pour prothèse de hanche, destinée à être enfouie dans le fémur à prothéser, se prolongeant par un col prothétique (5), dont l'extrémité est destinée à recevoir une tête destinée à coopérer avec un noyau de frottement lui-même reçu dans la cavité cotyloïdienne de l'os iliaque de l'articulation coxo-fémorale considérée, ***caractérisée* en ce que** la partie métaphysaire de la tige présente au niveau de sa partie antérieure, définie comme étant la face dirigée vers le devant du patient lorsque la prothèse est en place, une excroissance (10), propre à générer une asymétrie dans le plan sagittal, de telle sorte à davantage remplir et combler le défaut de matière osseuse à ce niveau, lors de la mise place de la tige.

2. Tige fémorale pour prothèse de hanche selon la revendication 1, ***caractérisée* en ce que** l'excroissance (10) va en s'élargissant d'une part de la zone métaphyso-diaphysaire en direction de la zone épiphysaire de la tige, et d'autre part de la zone corticale interne vers la face externe (8) de ladite tige.

3. Tige fémorale pour prothèse de hanche selon l'une des revendications 1 et 2, ***caractérisée* en ce que** la tige présente au niveau de sa face externe (8) une angulation, dont le sommet (4) est aligné avec l'axe P du col prothétique.

4. Tige fémorale pour prothèse de hanche selon l'une des revendications 1 à 3, ***caractérisée* en ce que** le col (5) de la tige présente une antéversion de l'ordre de 8°, résultant d'une hélitorsion progressive de la partie métaphysaire de ladite tige à partir de la jonction métaphyso-diaphysaire.

5. Tige fémorale pour prothèse de hanche selon l'une des revendications 1 à 4, ***caractérisée* en ce que** la tige (2) présente une courbure dans le plan sagittal, s'étendant sensiblement de la jonction métaphyso-diaphysaire en direction de la zone épiphysaire de la tige.

6. Tige fémorale pour prothèse de hanche selon l'une des revendications 1 à 5, ***caractérisée* en ce que** la tige présente sur chacune de ses deux faces principales des rainures (9), non débouchantes au niveau de l'extrémité supérieure de la zone proximale (3), et se terminant à leur extrémité inférieure par un rayon de courbure au moins égal à 1200 mm, de telle sorte à faciliter l'extraction de la tige hors du fémur le cas échéant.

7. Tige fémorale pour prothèse de hanche selon la revendication 6, ***caractérisée* en ce que** les rainures (9) sont de profondeur constante sur toute leur longueur, à l'exception de leur extrémité inférieure.

8. Tige fémorale pour prothèse de hanche selon l'une des revendications 1 à 7, ***caractérisée* en ce que** le col prothétique (5) présente à sa base deux plats latéraux symétriques (6), destinés à augmenter le débattement angulaire de la tige.

9. Tige fémorale pour prothèse de hanche selon la revendication 8, ***caractérisée* en ce que** le col prothétique (5) présente au niveau de sa jonction avec les deux plats latéraux symétriques (6) un rétreint (13).

## Claims

1. Femoral stem (1) for a hip prosthesis, which stem is intended to be embedded in the femur to be fitted with the prosthesis and is continued by a prosthesis neck (5) whose end is intended to receive a head intended to cooperate with a friction core which is itself received in the acetabular cavity of the iliac bone of the coxofemoral joint in question, **characterized in that** the metaphyseal part of the stem has, in its anterior part, defined as being the face directed towards the front of the patient when the prosthesis is in place, a protrusion (10) which generates an asymmetry in the sagittal plane in such a way as to better fill and make up for the absence of bone material at this level upon fitting of the stem.

2. Femoral stem for a hip prosthesis according to Claim 1, **characterized in that** the protrusion (10) widens, on the. one hand, from the metaphyso-diaphyseal zone in the direction of the epiphyseal zone of the stem, and, on the other hand, from the inner cortical zone towards the outer face (8) of said stem.

3. Femoral stem for a hip prosthesis according to one of Claims 1 and 2, **characterized in that** the stem has, on its outer face (8), an angulation whose summit (4) is aligned with the axis P of the prosthesis neck.

4. Femoral stem for a hip prosthesis according to one of Claims 1 to 3, **characterized in that** the neck (5) of the stem has an anteversion of the order of 8°, resulting from a progressive helitorsion of the metaphyseal part of said stem starting from the metaphyso-diaphyseal junction.

5. Femoral stem for a hip prosthesis according to one of Claims 1 to 4, **characterized in that** the stem (2) has a curvature in the sagittal plane, extending substantially from the metaphyso-diaphyseal junction in the direction of the epiphyseal zone of the stem.

6. Femoral stem for a hip prosthesis according to one of Claims 1 to 5, **characterized in that** the stem has, on each of its two main faces, grooves (9) which do not open out at the upper end of the proximal zone (3) and which, at their lower end, terminate in a radius of curvature at least equal to 1200 mm, in such a way as to facilitate removal of the stem from the femur if the need arises.

7. Femoral stem for a hip prosthesis according to Claim 6, **characterized in that** the grooves (9) have a constant depth along their entire length, except at their lower end.

8. Femoral stem for a hip prosthesis according to one of Claims 1 to 7, **characterized in that** the prosthesis neck (5) has, at its base, two symmetrical lateral flat areas (6) intended to increase the angular clearance of the stem.

9. Femoral stem for a hip prosthesis according to Claim 8, **characterized in that** the prosthesis neck (5) has a narrowing (13) in the area of its junction with the two symmetrical lateral flat areas (6).

## Patentansprüche

1. Femoraler Schaft (1) für eine Hüftprothese, der dazu bestimmt ist, in den mit der Prothese zu versehenen Oberschenkelknochen eingesetzt zu werden und der durch einen Prothesenhals (5) verlängert ist, dessen Ende zur Aufnahme eines Kopfes bestimmt ist, der zum Zusammenwirken mit einem Gleiteinsatz bestimmt ist, welcher seinerseits in der Gelenkpfanne des Hüftbeins des betroffenen Hüftgelenks aufgenommen ist, **dadurch gekennzeichnet, daß** der metaphysäre Abschnitt des Schaftes im Bereich seines anterioren Abschnittes, der als die zur Vorderseite des Patienten gerichtete Fläche im eingesetzten Zustand der Prothese definiert ist, einen Vorsprung (10) aufweist, geeignet zur Erzeugung einer Asymmetrie in der Sagittalebene, so daß das in diesem Bereich fehlende Knochenmaterial beim Anbringen des Schaftes besser aufgefüllt wird.

2. Femoraler Schaft für eine Hüftprothese nach Anspruch 1, **dadurch gekennzeichnet, daß** der Vorsprung (10) sich zum einen ausgehend von der metaphyso-diaphysären Zone in Richtung der epiphysären Zone des Schaftes und zum anderen von der inneren Corticalis zur Außenfläche (8) des genannten Schaftes vergrößert.

3. Femoraler Schaft für eine Hüftprothese nach einem der Ansprüche 1 und **2, dadurch gekennzeichnet, daß** der Schaft im Bereich seiner Außenfläche (8) eine Abwinklung aufweist, deren Scheitel (4) zur Achse P des Prothesenhalses ausgerichtet ist.

4. Femoraler Schaft für eine Hüftprothese nach einem der Ansprüche 1 bis 3, **dadurch gekennzeichnet, daß** der Hals (5) des Schaftes eine Anteversion der Größenordnung von 8° aufweist, die aus einer progressiven schraubenförmigen Windung (Helitorsion) des metaphysären Abschnittes des genannten Schaftes ausgehend vom metaphyso-diaphysären Übergang resultiert.

5. Femoraler Schaft für eine Hüftprothese nach einem der Ansprüche 1 bis 4, **dadurch gekennzeichnet, daß** der Schaft eine Krümmung in der Sagittalebene aufweist, welche sich im wesentlichen vom metaphyso-diaphysären Übergang in Richtung der epiphysären Zone des Schaftes erstreckt.

6. Femoraler Schaft für eine Hüftprothese nach einem der Ansprüche 1 bis 5, **dadurch gekennzeichnet, daß** der Schaft auf jeder seiner zwei Hauptflächen Nuten (9) aufweist, welche im Bereich des oberen Endes der proximalen Zone (3) nicht offen sind und an ihrem unteren Ende durch einen Krümmungsradius, der mindestens gleich 1.200 mm ist, enden, um gegebenenfalls das Herausziehen des Schaftes aus dem Oberschenkelknochen zu erleichtern.

7. Femoraler Schaft für eine Hüftprothese nach Anspruch 6, **dadurch gekennzeichnet, daß** die Nuten (9) eine konstante Tiefe über ihre gesamte Länge mit Ausnahme ihres unteren Endes aufweisen.

8. Femoraler Schaft für eine Hüftprothese nach einem der Ansprüche 1 bis **7, dadurch gekennzeichnet, daß** der Prothesenhals (5) an seiner Basis zwei symmetrische seitliche Flächen (6) aufweist, die zur Steigerung der Winkelfreiheit des Schaftes bestimmt sind.

9. Femoraler Schaft für eine Hüftprothese nach Anspruch 8, **dadurch gekennzeichnet, daß** der Prothesenhals (5) im Bereich seines Übergangs zu den zwei symmetrischen seitlichen Flächen (6) eine Einschnürung (13) aufweist.
